(19) Europäisches Patentamt
European Patent Office
Office européen des brevets

(11) **EP 0 696 446 A1**

## (12) EUROPEAN PATENT APPLICATION

(43) Date of publication:
**14.02.1996 Bulletin 1996/07**

(51) Int. Cl.$^6$: **A61F 2/06**

(21) Application number: **95112516.0**

(22) Date of filing: **09.08.1995**

(84) Designated Contracting States:
**CH DE FR GB LI**

(30) Priority: **09.08.1994 JP 187198/94**

(71) Applicant: **Olympus Optical Co., Ltd.**
**Tokyo (JP)**

(72) Inventor: **Miyano, Yasuo,**
**c/o Intellectual Property & Legal**
**Hachioji-shi, Tokyo (JP)**

(74) Representative: **KUHNEN, WACKER & PARTNER**
**D-85354 Freising (DE)**

(54) **Intraluminally indwelling stent and method for manufacturing the same**

(57) A stent has a material for generating an expansion retaining force at a lumen of interest of a human being and comprises a stent constituent element (2) with sections defining a space (4) therebetween and a film constituent element (3) attached to the stent constituent element (2) with the space covered and closed therewith. The film constituent element (3) is formed by dipping into a solution containing a material for the film constituent element (3). The thickness of the film constituent element (3) at those areas spread therewith but not at an area around each section of the stent constituent element (2) is equal to, or smaller than, the diameter of the sections of the stent constituent element (2).

F I G. 4

Printed by Rank Xerox (UK) Business Services
2.9.9/3.4

EP 0 696 446 A1

## Description

The present invention relates to a stent for use within extending/expanding the lumen of the respiratory, gastrointestinal and vascular system, the genital track, and so on and to a method for manufacturing the stent.

This type of intraluminally indwelling stent has often been applied particularly to the gastrointestinal system. This stent is used to extend and expand a narrowed or blocked lumen caused by a diseased region, such as a cancer and regain the luminal function originally possessed by the gastrointestinal tract or duct.

Conventionally, in order to recover from the narrowed or blocked gastrointestinal duct or tract involved, the usual practice is to surgically remove or repair this region. Recently, the treatment based on the internal medicine, such as the expanding, etc., of a balloon in the lumen of interest, is carried out without resecting it surgically.

The expansion of the lumen by a balloon, etc., has been temporarily done at a luminal tissue only during a medical procedure, sometimes again causing the narrowing or blocking of the lumen involved.

The procedure using an indwelling stent for expanding such a narrowed or blocked lumen of interest, either directly or with a balloon, etc., in a radial direction and retaining the lumen in an expanded state has been extensively carried out. JPN PAT APPLN KOKAI PUBLICATION 4-32662 discloses, as this type of stent, a stent made of a wire having no film constituent element.

It has been found that the stent made of a wire element only and adapted to expand the lumen involved and retain it in the expanded state has the drawback in that, due to a greater space or gap created between sections of the wire element, the neighboring tissue extends from the space into the stent and the stent is again narrowed or blocked.

The solution to this problem has been proposed by the specifications of U.S. Patent Nos. 4,776,337 and 5,282,824 according to which a film constituent element is added to a stent constituent element to provide an expansion retaining capability to the stent.

In this type of film-attached stent, it is necessary that the film constituent element have its own properties for producing the expansion capability and its retaining capability, for example, producing an expansion retaining force, a compressible force radially acting upon a surrounding tissue at a time of insertion and a returning force radially returning the stent back to its original state after insertion. Further, the film constituent element also need adequately prevent the passage of the stent from again being closed off or narrowed.

It is accordingly the object of the present invention to provide a stent comprised of an integral unit of a stent constituent element and film constituent element and adapted to allow the stent to be compressed upon being inserted into the lumen of interest of a human subject and the stent to be returned back to its original state, immediately after being inserted to the lumen involved, under an expansion retaining power of the stent constituent element and under less restrictive movement of the stent constituent element and adapted to prevent the passage of the stent in an indwelled state from being again blocked off or being narrowed, and a method for manufacturing a stent having a form of a film which can achieve the object of the present invention.

According to the present invention there are provided a stent and method for manufacturing the same as will be set out below.

In one aspect of the present invention, there is provided an intraluminally indwelling stent for use in a tract or duct of an internal system of a human subject, comprising:

a stent constituent element made of a material for imparting a substantially expansion retaining force, through a radial expansion, to the tract of interest and having a space between sections of the stent constituent element; and

a film constituent element so attached to the sections of the stent constituent element as one unit as to be spread across the sections of the stent constituent element with the space closed thereby, in which a thickness of the film constituent element at those areas spread therewith but not at an area around each section of the stent constituent element is equal to, or smaller than, a diameter of the sections of the stent constituent element.

The film constituent element can readily follow the movement of the basic stent constituent element.

According to another aspect of the present invention, there is provided a method for manufacturing a stent for use as an intraluminally indwelling stent in a duct or track of an internal system of a human subject, comprising the steps of:

(1) covering a stent constituent element with a solution containing a material for a film constituent element to form said film constituent element across a space defined between sections of the stent constituent element with the space blocked therewith; and
(2) removing a chemical, which is dissolved in the solution, from a film formed from the solution with the stent constituent element covered therewith and securing a physical property of the film constituent element itself.

In another aspect of the present invention, the method further comprises removing oil or grease from the surface of the sections of the stent constituent element and then performing the first and second steps (1) and (2).

The chemical should be removed from the film surface by the two steps: a first step for drying up the film surface by natural drying and a second step for drying up the film surface by virtue of heating. By the mere first step, the chemical cannot be adequately removed from the film constituent element so that a desired physical property is less likely to be obtained. By the second step only, the chemical is rapidly removed, thus causing a bubbling phenomenon to occur in the film constituent element. It is, therefore, not possible to obtain a desired physical property. The chemical is often irritant to the living body and the second step may desirably be effected under a vacuum level.

The solution may be coated on the stent constituent element by not only a dipping method but also a spraying, a brushing, etc.

As the material for the stent constituent element, use is made of a metal-based wire or a plastics-based wire. As the metal-based wire use is made of, for example, stainless steel. As a plastics-based wire, use is made of, for example, polyester. The material and diameter of the wire are selected, taking into consideration the outer diameter of an intended stent product and a requisite expansion force.

Further, in order to attain the intended object of the film constituent element without imparting a restriction to the stent constituent element, the thickness of the film constituent element is desirably made equal to, or smaller than, the outer diameter of the sections of the stent constituent element and, if the outer diameter of the stent constituent element is 1, the thickness of the film constituent element is more desirably of the order of 0.3 to 0.7.

This invention can be more fully understood from the following detailed description when taken in conjunction with the accompanying drawings, in which:

FIG. 1 is a side view showing a stent according to a first embodiment of the present invention with a film constituent element attached to a stent constituent element;

FIG. 2 is a side view showing the basic stent constituent element only;

FIG. 3 is a cross-sectional view showing a relation of the film constituent element to the basic stent constituent element;

FIG. 4 is a side view showing a variant of the stent in the first embodiment of the present invention;

FIG. 5 is a side view showing a basic stent constituent element alone in a second embodiment of the present invention; and

FIG. 6 is a side view showing a relation of a film constituent element to a stent constituent element.

The embodiments of the present invention will be explained below in more detail with reference to the accompanying drawings.

[First Embodiment]

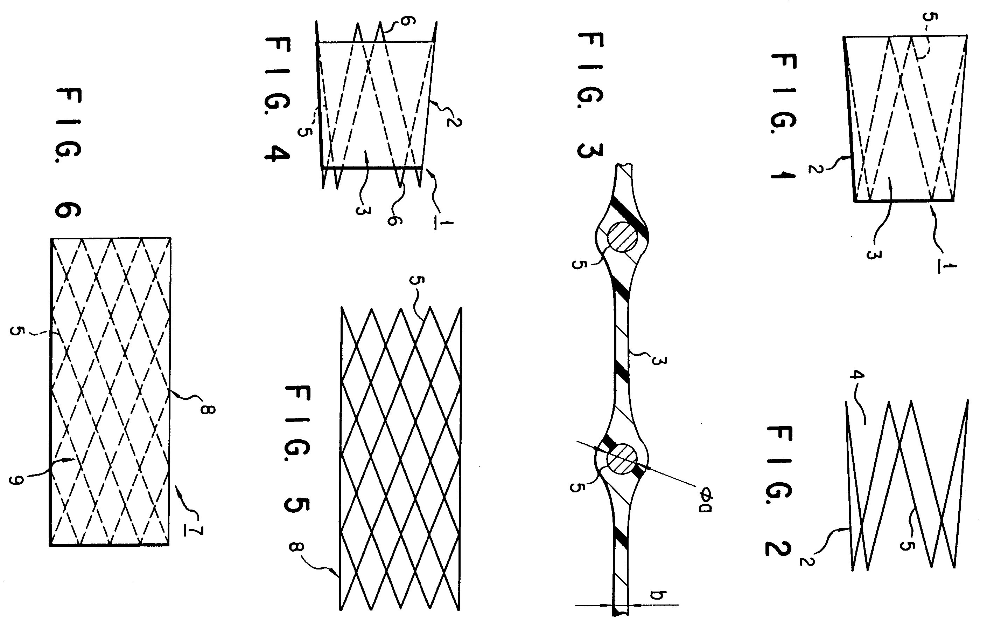

In FIG. 1, a stent according to a first embodiment of the present invention is used for the gastrointestinal duct or track of a human subject. Generally viewing the form of the stent 1, the stent is of such a substantially cylindrical configuration that it is somewhat tapered toward one end. The stent 1 comprises a basic stent constituent element 2 and a film constituent element 3 integrally attached to the basic stent constituent element. The stent 1 has an elastic power and a self-expanding capability for expanding itself in a radial direction. In order to impart an elastic expansibility to the stent, the basic stent constituent element 2 is comprised of, as shown in FIG. 2, a multi-bent stainless wire whose imarginary envelope constitutes an annular or cylindrical configuration. That is, the wire 5 serving as a skeleton is comprised of an imaginary cylindrical envelope where it is multi-bent along its periphery. The wire has its ends joined together to provide a closed loop. As a material for the stent constituent element 2 use is made of a wire normally 0.2 mm in diameter. The basic stent constituent element 2 is 15 mm in outer diameter and 30 mm in full length in its free state. This can be used in the gastrointestinal duct or tract. It is readily appreciated that, needless to say, the material and diameter of the wire 5 in the basic stent constituent element 2, as well as the outer diameter and full length of the stent 1, can be properly varied and selected, without departing from an intended range of the present invention, taking various factors into consideration.

Between the sections of the wire 5, that is a material of the basic stent constituent element 2, a gap or space 4 is defined as shown in FIG. 2. The film constituent element 3 is so provided as to close the gap or space. The film constituent element 3 is attached as one unit to the wire 5 and spread as an integral unit.

The manufacturing method of the present invention will be explained below.

First, in order to enhance the adhesion of the film constituent element 3 to the wire 2a in the basic stent constituent element 2 and achieve stability, the wire 2a was degreased with acetone. In the degreasing step, an acetone solution was prepared in an amount enough great to immerse the basic stent constituent element 2 therein. The basic stent constituent element 2 was immersed in the acetone solution and subjected to ultrasonic cleaning for five minutes. Then at room temperature the stent constituent element 2 was allowed to stand until the acetone was evaporated.

Then, the degreased stent constituent element 2 was dipped in a solution where the material for the film constituent element 3 was dissolved.

As shown in FIG. 3, a wire element 5 of the stent constituent element 2 was coated with a solution, while forming a film around its outer outline, and joined there.

After being dipped in the solution, the element 5 was allowed to stand at room temperature for 30 minutes and dried up at 60°C under a vacuum of $9.8 \times 10^{-4}$ Pa for three hours.

From the resultant film surface around the outer periphery of the wire element 5, a chemical in the solution was removed to secure the physical properties of the film constituent element 3 itself.

The film constituent element 3 was attached firmly around the wire element 5 of the stent constituent element 2 to provide a bond there so that the film constituent element 3 was hard to strip from the stent constituent element 2.

As the material for the film constituent element 3 use was made of a Shore D 70° polyurethane and as its solvent use was made of tetrahydrofuran (THF) and the concentration of the material for the film constituent element 3 was so prepared as to be 10%. The dipping was effected three times, taking into consideration the area of the stent space 4, that is, the space area provided between the sections of the wire element 5 in the stent constituent element 2, as well as a ratio of a/b = 1/2 between the outer diameter a of the wire element 5 in the stent constituent element 2 and the thickness b of the film constituent element 3, except around the wire element 5, as shown in FIG. 3. The basic stent constituent element was allowed to stand at room temperature for 10 minutes at each dipping.

In the above case, although the ratio is set to a:b = 2:1, it should not be restricted to that value. The thickness b of the film constituent element 3 may be properly varied depending upon the set concentration of the solution and, by doing so, the film thickness can be controlled.

As shown in FIG. 3, the film constituent element 3 provides a firm bond around the wire element 5 of the basic stent constituent element 2 and, through the ratio of a:b = 2:1 above, the thin film is formed between the sections of the wire element 5 at those areas where wire element 5 is present.

Although, in the first embodiment above, the film constituent element 3 covers the full length of the stent constituent element 2, uncovered areas 6 may be formed at least one-end side of the sections of the stent constituent element 2 as shown in FIG. 4.

[Second Embodiment]

A stent according to a second embodiment of the present invention will be explained below with reference to FIGS. 5 and 6.

This stent 7 comprises a stent constituent element 8 and film constituent element 9. The stent constituent element 8 is expanded by a means, such as an instrument having an expansion function different from the stent above, and has a function of retaining its expanded shape. As the wire element for the stent constituent element 8 use is made of, for example, stainless steel wires spirally wound in a mutually intersecting way to provide a generally cylindrical network structure with each mesh defined as a stent space.

The wire element of the stent constituent element 8 was manufactured to have a diameter of 0.5 mm and, in an expanded state, the stent was 15 mm in outer diameter and 30 mm in full length. The material and diameter of the stent constituent element 8 as well as the outer diameter and full length of the stent can be determined in the same way as in the first embodiment.

Though it may be considered that, with the stent constituent element 8 in an expanded, in a contracted and in a moderately expanded state the film can be formed, it is possible to use these three state according to the present invention. Here, an explanation will be given below of the formation of the film with the stent constituent element 8 in the expanded state. The other two manufacturing methods, not being described here, may be treated in a similar way as set out above.

A film is formed, in the same step as that in the first embodiment, around an expanded stent constituent element shown in FIG. 5 to provide a film constituent element 9 as shown in FIG. 6.

The second embodiment is different from the first embodiment in that a ratio is a:b = 1:1 where, in the same way as shown in FIG. 3, "a" represents the outer diameter of the wire element in the stent constituent element 8 and "b", the thickness of the film constituent element 9 at those areas other than the wire element. The reason is that, since the outer diameter of the stent constituent element 8 is smaller than that of the stent constituent element 2 in the first embodiment, the mechanical property of the film constituent element 9 is not adequate if a:b = 2:1.

The stent constituent element in each embodiment is made of a metal material, desirably stainless steel or tungsten. The stent constituent element may be made of a plastics material. As the plastics material, there are polyethylene, polyester, polyamide, liquid crystal polymer, etc.

As the material for the film constituent element, use may be made of an elastomer-series plastics material, such as polyurethane elastomer, polyester elastomer and polyamide elastomer. If this is the case, any displacement resulting from the contraction of the stent is less generated and, in this respect, it is desirable.

For the manufacture of this type of stent, it is necessary to adequately join together the stent constituent element and film constituent element from a practical viewpoint. As a method for providing a close bond between these elements, spraying by a sprayes, as well as dipping, may be considered, but the dipping method is most desirable to provide an ideal bond, because the film constituent element can be so formed as to surround or cover the outline of the wire element sections by which the network's meshes are defined. More desirably, a close bond-preventing material, such as a grease or oil, deposited on the stent constituent element prior to the dipping step, can be eliminated. It is considered that, as the chemical used at the degreasing step, there are, for example, a ketone compound such as acetone and MEK (methyethylketone), alcohols such as methanol and ethanol, an organic halogen compound such as trichloroethylene and carbon tetrachloride, an aromatic compound such as benzene, toluene and THF (tetrahydrofran), and a sulfur compound such as DMSO (dimethylsulfoxide). The use of these chemicals is restricted depending upon their degreasing capability and resistance of the stent constituent element to the chemicals. Further, ultrasound desirably may be used together with the chemicals.

The number of times the dipping is done for the formation of the film constituent element is adjusted by the diameter of the stent constituent element, distance between adjacent sections of the stent constituent element, concentration of a solution used and thickness of the film intended.

The dipping solution is comprised of chemicals by which the material for the film constituent element is solvable. The concentration of the solution is 0.5% to 50%, desirably 5% to 20%. As the material for the film constituent element, it is desirable to use, for example, a thermoplastic elastomer (hereinafter referred to as TPE) and having, as a hardness, about Shore A 30° to Shore D 95° and having a combination with disolvable chemicals. A typical combination of TEP with the solvable chemical is an urethane-series TPE and THF (tetrahydrofuran), and a polyamide-series TPE and DMSO (dimethylsulfoxide), and so on.

The present invention is not restricted to the above-mentioned embodiments and various changes or modifications may be made without departing from the spirit and scope of the present invention.

**Claims**

1. A stent for use in a lumen of an internal system of a human subject, characterized by comprising:
a basic stent constituent element (2) made of a material for imparting an expansion retaining force to the lumen of interest and having a space (4) between adjacent sections of the stent constituent element; and a film constituent element (3) so attached to the sections of the stent constituent element (2) as to be spread across the sections of the stent constituent element (2) with the space (4) closed thereby, in which a thickness of the film constituent element (3) at those areas covered therewith but not at the area around each section of the stent constituent element (2) is equal to, or smaller than, a diameter of the sections of the stent constituent element (2).

2. The stent according to claim 1, characterized in that the film constituent element (3) is joined integral with the sections of the stent constituent element.

3. The stent according to claim 1, characterized in that the film constituent element (3) is so formed as to surround an outline of the sections of the stent constituent element.

4. The stent according to claim 1, characterized in that the stent constituent element (2) has a substantially annular envelope configuration and is radially so expandable as to substantially generate an expansion retaining force at the lumen of interest.

5. The stent according to claim 1, characterized in that the stent constituent element (2) has a tapered tubular configuration and is radially so expandable as to substantially generate an expansion retaining force at the lumen of interest.

6. The stent according to claim 1, characterized in that the stent constituent element (2) is made of a metal material, preferably comprised of stainless steel or of tungsten.

7. The stent according to claim 1, characterized in that the stent constituent element (2) is made of plastics material, preferably made of polyethylene or comprised of polyester or comprised by polyamide, or a liquid-crystal polymer.

8. The stent according to claim 1, characterized in that the film constituent element (2) is made of an elastomer-series plastics material, preferably comprised by polyurethane elastomer, or comprised by polyester elastomer, or comprised of polyamide elastomer.

9. A method for manufacturing a stent for use as a stent in a lumen of an internal system of a human subject, characterized by comprising the steps of:

(1) coating a basic stent constituent element (2) with a solution containing a material for a film constituent element (3) to form said film constituent element (3) across a space defined between sections of the stent constituent element (2) with the space covered therewith; and (2) removing a chemical, which is dissolved in the solution, from a film formed from the solution with the stent constituent element (2) coated therewith and securing a physical property of the film constituent element (3) itself.

10. The method according to claim 9, characterized by further comprising removing oil or grease from a surface of the sections of the stent constituent element (2) and then performing the first and second steps.

11. The method according to claim 9, characterized in that said first step is performed by dipping the stent constituent element (2).

12. The method according to claim 9, characterized in that the first step is done by coating the solution by a brush on the stent constituent element (2).

13. The method according to claim 9, characterized in that the first step is done by spraying the solution on the stent constituent element (2).

14. The method according to claim 9, characterized in that the stent constituent element (2) is made of a metal material, preferably comprised of stainless steel or comprised of tungsten.

15. The method according to claim 9, characterized in that the stent constituent element (2) is made of plastics material, perferably made of polyethylene, or comprised of polyester, or comprised of polyamide, or a liquid-crystal polymer.

16. The method according to claim 9, characterized in that the film constituent element (3) is made of an elastomer-series plastics material, preferably comprised of polyurethane elastomer, or comprised of polyester elastomer, or comprised of polyamide elastomer.

FIG. 1

FIG. 2

FIG. 3

FIG. 4

FIG. 5

FIG. 6

**European Patent Office**    **EUROPEAN SEARCH REPORT**    Application Number

EP 95 11 2516

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FR-A-2 688 688 (T. RICHARD ET AL.)<br><br>* page 2, line 10 - page 3, line 19; figures 1,2,7 *<br>* page 4, line 30 - page 5, line 2 *<br>--- | 1-11, 14-16 | A61F2/06 |
| X | DE-A-39 18 736 (C. VALLBRACHT)<br><br>* column 2, line 10 - line 12 *<br>* column 2, line 24 - line 29 *<br>--- | 1-4,6-9, 11,14-16 | |
| P,X | WO-A-94 24961 (SCHNEIDER (USA) INC.)<br><br>* page 8, line 4 - page 10, line 28; claims 3,5; figures 1-7B *<br>--- | 1-11, 13-16 | |
| P,X | EP-A-0 621 015 (SCHNEIDER (EUROPE) AG)<br><br>* column 2, line 25 - column 3, line 34; figures *<br>--- | 1-9,11, 14-16 | |
| D,X | US-A-4 776 337 (J.C. PALMAZ)<br><br>* column 6, line 20 - line 38; figures 1A,1B *<br>* column 9, line 24 - line 34; figures 5,6 *<br>----- | 1,2,4, 6-8 | **TECHNICAL FIELDS SEARCHED** (Int.Cl.6)<br><br>A61F |

The present search report has been drawn up for all claims

| Place of search | Date of completion of the search | Examiner |
|---|---|---|
| THE HAGUE | 17 November 1995 | Wolf, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)